# EUROPEAN PATENT APPLICATION

(11) **EP 4 729 937 A1**
(43) Date of publication of application: **22.04.2026**
(21) Application number: 25207672.4
(22) Date of filing: 09.10.2025
(51) Int. Cl.: G01N 33/46, G06F 30/20, G06F 119/18

(54) **COMPUTER-IMPLEMENTED METHOD FOR PREDICTING A STRENGTH GRADE OF A WOODEN BOARD BEFORE CUTTING IT AND USES OF THAT METHOD**

(30) Priority: 17.10.2024 IT 202400023127
(71) Applicant: MICROTEC S.p.A., 39042 Bressanone (BZ) (IT)
(72) Inventor: BACHER, Martin, 39030 PFALZEN BZ (IT); URSELLA, Enrico, 30174 MESTRE VE (IT)
(74) Representative: Ponchiroli, Simone

(57) **Abstract**

A computer-implemented method for predicting a real strength grade which a known grading device (7) will assign to a real wooden board (2) not yet cut from a log (6), comprising a virtual cutting step of a three-dimensional virtual model (1) of the log (6) to obtain a virtual board (4), an assigning step, during which surface and/or internal characteristics are added to the virtual board (4), a virtual grading step, during which a virtual strength grade which the known grading device (7) would assign to the virtual board (4) is estimated, a predicting step, during which it is assumed that the real strength grade, which the known grading device (7) will assign to the real wooden board (2), corresponds to the virtual strength grade estimated in the virtual grading step.

## Description

This invention relates to a computer-implemented method for predicting a strength grade of a wooden board before cutting it, as well as some possible uses of that method.

Strength grading is an assessment performed on wooden boards intended for structural applications, in particular for making lamellar beams (that is to say, beams consisting of a plurality of staggered overlapping boards glued to each other). At European level strength grading is defined by standard **EN** 14081 and the strength classes by standard **EN** 338.

As can be easily inferred, in the context of grading of boards intended to be used for structural applications, it is possible to proceed with their strength grading exclusively with non-destructive testing (preferably also such that the boards themselves are not subjected to any type of mechanical stress).

The need to be able to supply a strength grade led to the development, over the years, of various certified apparatuses intended to assess the strength grade of wooden boards.

However, as is clearly explained in the article by Bacher, Martin, "Comparison of different machine strength grading principles", COST E53 Conference 29th-30th October, Delft, The Netherlands, 2008 ([1]), all of the non-destructive techniques available at industrial level for strength grading wooden boards have relatively low reliability, consequently even certified grading devices, usually used for that purpose, have a strength grading reliability which may only equal around 80%. However, in the sector, that assessment reliability is considered statistically sufficient, given that as a whole there is a limit on both the number of potentially good boards which are nevertheless rejected, and the number of boards potentially to be rejected which are nevertheless deemed to be good (with regard to this aspect consider that structural boards often are not intended to be used individually but together with many others in lamellar beams).

As a result of the introduction of strength certifications, log cutting plant operators have taken a particular interest in succeeding in cutting logs in such a way as to obtain the best possible boards in terms of mechanical strength, in order to optimise the economic return on the timber.

In light of that, the doctoral thesis by Brännström, Mattias, "Integrated strength grading" Diss. Luleå Tekniska Universitet, 2009 ([2]), showed that it is possible to generate images containing the characteristics of a virtual board obtained from a log in order to estimate its strength. However, it was possible to verify that the reliability of such a system is potentially similar to that of an automatic grading device and that the false positives and false negatives in the two systems are generally different (boards considered good in the tomographic analysis may be rejected by the grading device), with the consequence that performing a preliminary analysis of this type does not help to notably increase the efficiency of a production system.

Therefore the prior art technology has important disadvantages.

Therefore, as seen, not only do the certifying machines have relatively low reliability, but also the techniques for estimating the strength class of a board before proceeding with cutting it allow little improvement in the overall efficiency of the plant.

In this context, the technical purpose which forms the basis of this invention is to at least partly overcome the above-mentioned disadvantages.

In particular the technical purpose of this invention is to define a technical solution which allows a significant increase in the overall efficiency of a plant, that is to say, the logs being equal, allows the obtainment of a greater number of boards which the grading device can grade as boards for structural use.

The technical purpose specified and the aims indicated are substantially achieved thanks to the definition of a computer-implemented method for predicting a strength grade of a wooden board before cutting it, in accordance with what is set out in independent claim 1. Particular embodiments of this invention and its particular applications are defined in the other claims.

This invention is based on a change in viewpoint concerning what was previously known and suggested by studies in the sector.

Whilst up until now there was an attempt to make assessments of the strength class performed beforehand and afterwards on various boards absolutely more precise, with the aim of being able to perfectly grade 100% of boards, in accordance with this invention there is no longer an attempt to maximise the absolute reliability of the two different assessments, the focus instead being on making assessments carried out beforehand as close a match as possible to those performed afterwards. In other words, in accordance with this invention, before cutting a log the aim is not to assess the effective strength class of the boards which can be obtained (that is to say, the strength class which could be measured by carrying out a destructive test), but instead action is taken to predict how each board which can be obtained from the log would be graded by the grading device effectively present in the relevant plant.

In fact, in this way, it is possible to notably increase the reliability of the entire system, making it substantially equal to that of only the grading device.

Further features and the advantages of this invention will be more apparent in the detailed description of several preferred, non-limiting embodiments of a computer-implemented method for predicting a strength grade of a wooden board before cutting it and some uses of that method illustrated with reference to the accompanying drawings, in which:
- Figure 1 is a schematic perspective view of a three-dimensional virtual model of a log inside which a knot is present;
- Figure 2 shows the application of a known cutting pattern to the three-dimensional virtual model of Figure 1, to obtain a virtual board;
- Figure 3 shows taking a virtual X-ray of the virtual board of Figure 2;
- Figure 4 shows the taking a real X-ray of a real board which corresponds to the virtual board of Figure 3;
- Figure 5 shows an example of a virtual X-ray taken of a virtual board;
- Figure 6 shows an example of a real X-ray taken of a real board which corresponds to the virtual board of Figure 5;
- Figure 7 is a block diagram of the computer-implemented method for predicting a real strength grade according to this invention;
- Figure 8 is a block diagram of an embodiment of a step of obtaining a three-dimensional virtual model of a log, part of the method of Figure 7; and

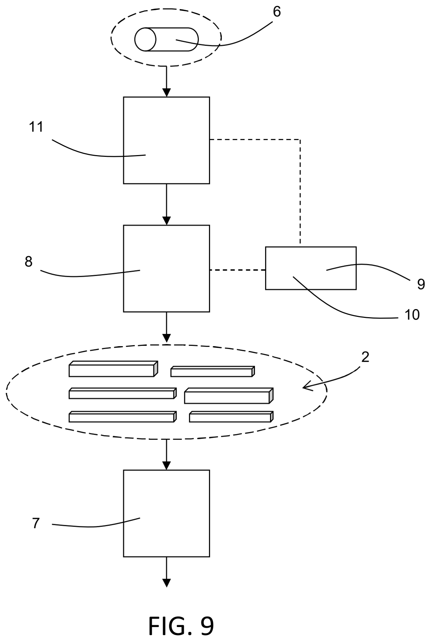

- Figure 9 schematically illustrates a plant for cutting logs into boards which incorporates this invention.

The main innovative aspect of this invention is the definition of a computer-implemented method for predicting a real strength grade which a known grading device will assign to a real wooden board, before the real wooden board is cut from a log 6 using a known cutting pattern.

As is known, each log 6 extends along a longitudinal axis and a cutting pattern is configured to divide the log 6 into real boards each having an end face, which lies in a plane transversal to the longitudinal axis, and lateral faces which are substantially parallel to the longitudinal axis (except for small inclinations which may commonly be present). In fact a cutting pattern comprises (and is advantageously defined by) a set of cutting planes substantially parallel to the longitudinal axis whose positions relative to each other and relative to the log 6 are defined; moreover, generally, the cutting planes of a cutting pattern are divided into two groups, with all of the cutting planes of one group parallel to each other and perpendicular to the cutting planes of the other group. By making all of the cuts required by the cutting pattern on the log 6 a plurality of real wooden boards is obtained.

The method which forms the main innovative aspect of this invention is intended not to attempt to calculate the real strength of the board, as occurs in the prior art, but to predict how a known grading device would grade a real board if it were obtained from a specific known part of a log 6 (that is to say, the part of the log 6 located between the cutting planes which, once executed, define the lateral faces of the real board).

This being a computer-implemented method, it shall be understood that all of the steps of which it is comprised, and which are described below, may be carried out by one or more computers, and in particular by a data processing device comprising a hardware infrastructure to which one or more computers belong (which must be programmed to implement the method).

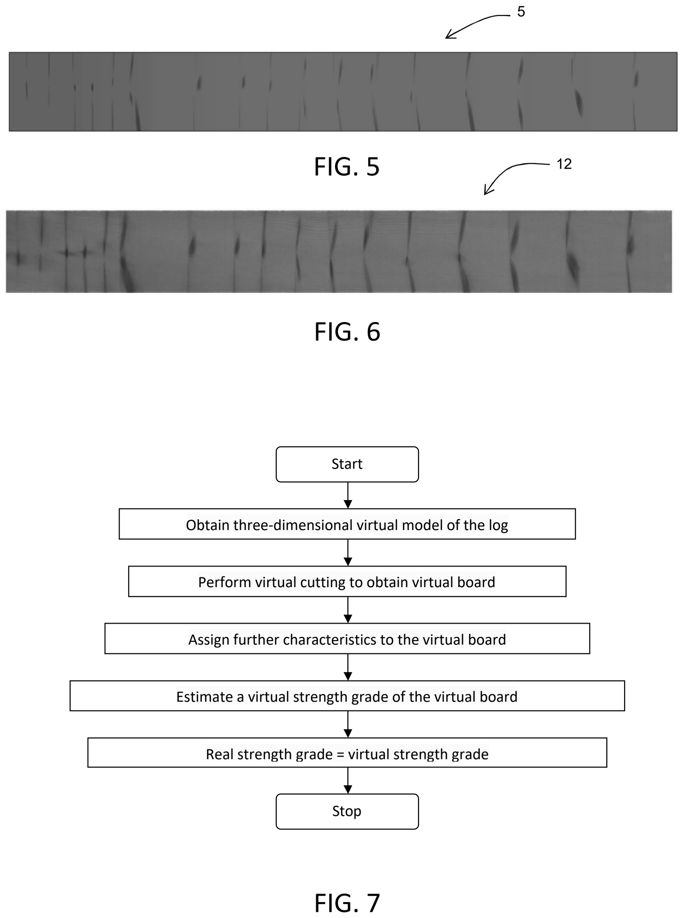

Figure 7 is a block diagram of the method.

The starting point for executing the method according to this invention is to prepare a three-dimensional virtual model 1 of the log 6 from which the real wooden board 2 must be obtained (it may be any of those obtainable with the known cutting pattern - hereinafter also indicated as board 2 or real board 2). Depending on the embodiments, the three-dimensional virtual model 1 may have different characteristics, and may comprise more or less details about the characteristics of the wood of which the log 6 consists (for example about the presence of knots 3).

In some embodiments, the three-dimensional virtual model 1 is already available at the start of execution of the method.

In some embodiments, the three-dimensional virtual model 1 is not available at the start of execution of the method; in those cases, the method may comprise a virtualisation step, during which the three-dimensional virtual model 1 of the log 6 is obtained. In some embodiments the virtualisation step is carried out by creating the three-dimensional virtual model 1 of the log 6 using digitally saved information about geometric characteristics of the log 6. Advantageously, the digitally saved information about the geometric characteristics of the log 6 comprise one or more of the following: a tomographic model of the log 6 and a three-dimensional geometric model of the outer shape of the log 6. In other embodiments, in contrast, the virtualisation step is carried out by retrieving the three-dimensional virtual model 1 of the log 6 previously digitally saved.

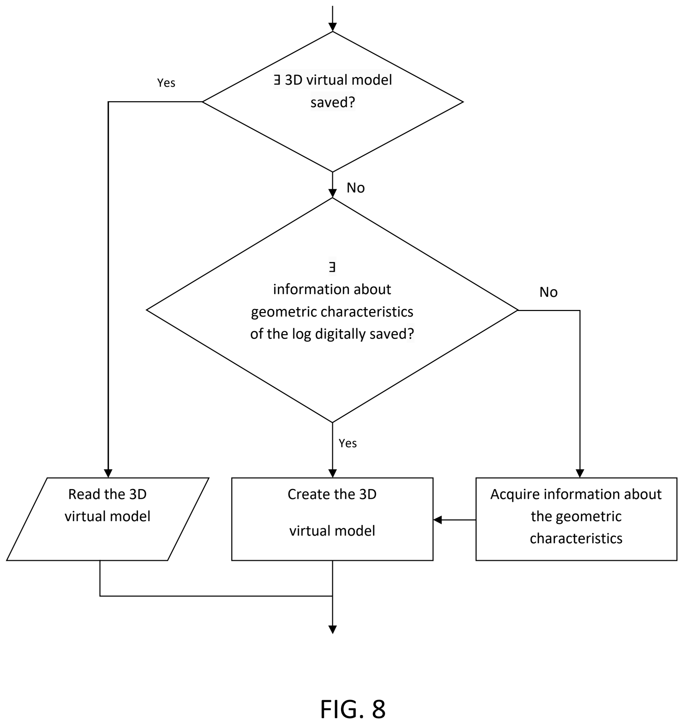

Figure 8 shows what was just described in the form of a block diagram.

Once the three-dimensional virtual model 1 is available, and a known cutting pattern to be used has been identified (known, in the sense that the number and positions relative to each other of the various cutting planes, and the absolute orientation relative to the log 6 are known), the method comprises execution of a virtual cutting step, during which the three-dimensional virtual model 1 of the log 6 is virtually cut by applying the known cutting pattern to the three-dimensional virtual model 1. For each cut the thickness of the blade intended to make it and the consequent thickness of the wood which is lost during the cutting are also taken into consideration. In this way a virtual board 4 is obtained which corresponds to the real wooden board 2 to be cut, that is to say, which in the three-dimensional virtual model 1 occupies the same position that the real board 2 occupies in the log 6. No further details will be provided about the ways in which a virtual cutting step of a virtual model of a log 6 is executed, since this is a widely known aspect to people who are experts in the sector.

At a software level, the virtual board 4 may also be defined by the position of its four lateral faces inside the three-dimensional virtual model 1 of the log 6 and it may comprise the part of the three-dimensional virtual model 1 enclosed by them.

The method then comprises execution of an assigning step, of virtually adding to the virtual board 4 surface and/or internal characteristics, obtaining them from digitally saved information about the log 6. In some embodiments, the assigning step is carried out after the virtual cutting step, whilst in other embodiments the two steps are carried out substantially in parallel. The latter case occurs in particular when the three-dimensional virtual model 1 of the log 6 comprises surface and/or internal characteristics of the log 6 from which the surface and/or internal characteristics of the virtual board 4 are obtained (for example by cutting with the cutting planes any knots 3 present in the three-dimensional virtual model 1).

In some embodiments, the digitally saved information comprises one or more of the following: a tomographic model of the log 6; one or more X-ray views of the log 6; a three-dimensional geometric model of the outer shape of the log 6; a map of the colour of an outer lateral surface of the log 6. Each of these types of digitally saved information may be used to identify some of the main characteristics of the log 6, which in this way can be assigned to the three-dimensional virtual model 1 and/or to the virtual board 4. In particular, each of them allows identification of the presence and the position of at least the main knots 3 of the log 6. That this is possible starting with a tomographic model or one or more X-ray views is widely known: see for example Giovannini S., Boschetto D., Vicario E., Cossi M., Busatto M., Ghidoni S., Ursella E., "Improving knot segmentation using Deep Learning techniques", 21st International Nondestructive Testing and Evaluation of Wood Symposium, September 24-27, 2019, Freiburg, Baden-Württemberg, Germany ([3]). A similar result can also be obtained starting with knowledge of the three-dimensional geometric model of the outer shape of the log 6 or of a colour map of an outer lateral surface of the log 6, given that knowledge of at least one of these aspects allows precise identification of every knot 3 on the outer surface of the log 6 and given that the ways in which a knot 3 grows inside a log 6 can be estimated with good precision: see for example Zolotarev, Fedor, et al., "Modelling internal knot distribution using external log features", Computers and Electronics in Agriculture 179 (2020): 105795 ([4]), and Habite, Tadios Sisay, and Anders Olsson, "Modelling of Knots and 3d Fibre Orientation within Timber Boards Based on Information Obtained from Optical Scanning", SSRN 4110671.

In some embodiments, in contrast, knowledge of the tomographic model or of the colour map of the outer lateral surface of the log 6 allows estimation of the direction of the fibre on the surface of the virtual board 4: see for example [3], Ursella, Enrico, "In-line industrial computed tomography applications and developments", Final doctoral thesis, (2021) ([5]), Longuetaud, Fleur, et al. "Automatic detection of pith on CT images of spruce logs", Computers and Electronics in Agriculture 44.2 (2004): 107-119 ([6]), and Habite, Tadios Sisay, and Anders Olsson, "Modelling of Knots and 3d Fibre Orientation within Timber Boards Based on Information Obtained from Optical Scanning", SSRN 4110671 ([7]).

In some embodiments, the digitally saved information comprises, in addition to what was indicated above, a value of a resonance frequency of the log 6 as an estimate of the modulus of elasticity of the wood (which will have been previously measured with the normal techniques used for that purpose).

Once the virtual cutting and assigning steps are complete, and therefore once all relevant information has been added to the virtual board 4, the method comprises a virtual grading step, during which an estimate is made of which virtual strength grade the known grading device would assign to a real board 2 if it were to correspond to the virtual board 4 obtained at the end of the assigning step (that is to say, if it were to have the same characteristics as the virtual board 4).

When the virtual grading step has ended, the method comprises a predicting step during which the virtual strength grade, estimated in the virtual grading step, is matched to the real strength grade which the known grading device will assign to the real wooden board 2.

Depending on the embodiments, the virtual grading step may be carried out using various types of algorithm.

In some embodiments, the virtual grading step is carried out using an algorithm which correlates characteristics of the virtual board 4 with the virtual strength grade. In this case, the characteristics of the board taken into consideration by the algorithm may be either all of the characteristics of the virtual board 4 or, more reasonably, only some of them, such as those relating to the knots 3, those relating to the trend of the direction of the fibres, those relating to the outer appearance of the lateral faces (which in turn is linked to the presence of knots 3 and/or to the surface trend of the direction of the fibres), etc.

In some embodiments, in particular, the algorithm used may be a neural network or a deep neural network, which uses data about the characteristics of the virtual board 4 as input data and which supplies the virtual strength grade as the output data item. A neural network or a deep neural network of this type may be particularly advantageous where it is possible to have a large amount of data to use as training data. For example, a neural network or a deep neural network of this type may advantageously be trained using the data normally supplied by log cutting plants in which a grading device of the relevant type is present. In fact, the presence of the grading device guarantees knowledge of the expected output for each board processed by the plant; moreover, in most plants also available (in digital format) or directly are the virtual board 4 which corresponds to each real board 2 (for example when the cutting pattern has been selected as a result of an analysis of the internal structure of the log 6), or data which in any case allow its reconstruction afterwards. Therefore, the preferred training method is carried out using the data generated and/or managed during normal operation of the plant.

Returning to the virtual grading step, in other embodiments it is carried out by simulating grading of the virtual board 4 by the known grading device. In this case, advantageously, the virtual grading step comprises generating for the virtual board 4 the same type of information that the grading device uses (advantageously after having generated it) for the real boards, and processing that information with the same algorithm also used by the grading device.

In more detail, the virtual grading step comprises a preparing step and a processing step. During the preparing step, for the virtual board 4, a set of virtual information to be assessed is obtained, where the virtual information to be assessed is of the same type as the real information to be assessed based on which the known grading device determines the real strength grade for a real board 2. During the processing step, the set of virtual information to be assessed is processed with the processing algorithm used by the known grading device for grading the strength of the real boards starting with the set of real information to be assessed.

In some embodiments, during the preparing step, for the virtual board 4, a set of virtual information to be assessed is generated which comprises one or more virtual X-ray images of the virtual board 4, advantageously obtained by simulating taking one or more X-rays of the virtual board 4.

In some embodiments, during the preparing step, for the virtual board 4, a set of virtual information to be assessed is generated which comprises one or more virtual maps which show a trend of the direction of the fibres of the wood at one or more faces of the virtual board 4.

In some embodiments, the set of virtual information to be assessed also comprises a value linked to a resonance frequency of the log 6.

Preferably, each set of virtual information to be assessed is generated using the above-mentioned digitally saved information.

It should be noticed that the different types of sets of virtual information to be assessed just described, to be used for executing the processing step, may also be used as input data in the embodiments in which the virtual grading step is carried out by means of a neural network or a deep neural network.

Below are some example embodiments of what was described so far.

### Example 1

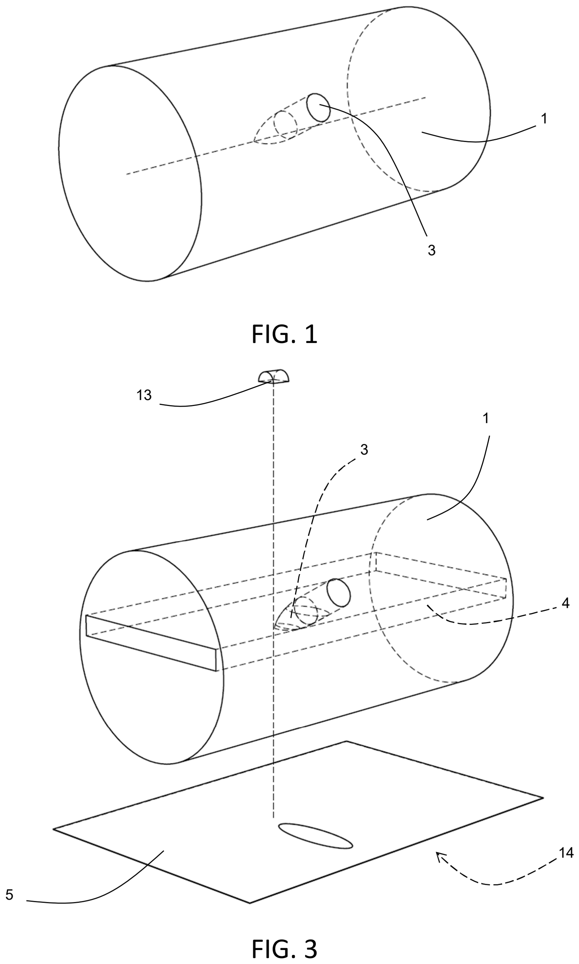

By carrying out a tomographic scan of the log 6 it is possible (see [3]) to identify the position, the size and the status (sound/dead) of the knots 3 in the log 6 (Figure 1).

The density of the wood of which the knots 3 consist may be assumed to be equal to a constant value (approximately 1 kg/dm³) or estimated on the basis of the tomographic image itself.

The density of the sound wood in many conifers may be deduced from the density of the heartwood of the fresh log 6 (easily measurable with tomography) reduced by 20% to take into account the fact that the fresh heartwood has a typical 30% moisture content whilst the final boards, after drying, have a moisture content of 12%.

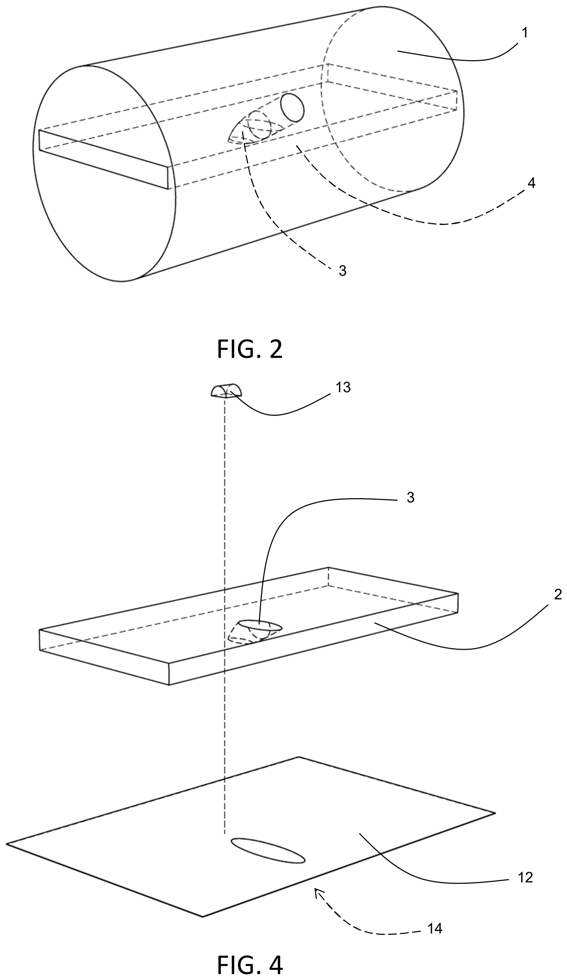

Once the known cutting pattern has been applied on the three-dimensional virtual model 1 of the log 6 (and the virtual board 4 obtained - Figure 2), it is then possible to know the density of every point of the virtual board 4 which corresponds to the relevant real board 2. Therefore, in this case, the virtual cutting step and the assigning step are carried out simultaneously.

At this point, knowing the position of the source 13 and of the sensors 14 of an X-ray scanning system belonging to the known grading device, it is possible to calculate the signal which would be measured by the sensor if the virtual board 4 were inserted into the known grading device (Figure 3), considering that it depends on the total density through which the X-rays pass and taking into account the Beer-Lambert law.

Therefore a virtual X-ray image 5 of the virtual board 4 can be produced (Figure 3). However, an X-ray image of the board corresponds to the typical input of some grading devices certified for estimating the strength of boards. Therefore, it is possible to use the same algorithms normally used by the grading device with the simulated input of the virtual board 4 to obtain a precise prediction of the strength which would be estimated by the device for the corresponding real board 2 (Figure 4).

Figures 5 and 6 show respectively a virtual X-ray image 5 of a virtual board 4 and a real X-ray image 12 of the corresponding real board 2.

### Example 2

As already indicated, other types of grading devices are based on measurement of the direction of the fibre of the wood on the surface of the board. On a real board 2 the direction of the fibre may be measured by projecting laser light dots onto the surface of the board and detecting the direction in which the light scatters inside the wood.

The direction of the fibre in a tree is influenced by three main characteristics: the knots 3, the spiral grain and the pith.

These characteristics are extractable from tomographic images ([3],[5],[6]). [7] also explains a method for being able to predict the fibre on the surface of a board starting from the internal characteristics and its reliability is compared with a board scanner.

### Example 3

[4] explains a method for estimating the presence of knots 3 inside a board given the outer shape of the log 6 and the cutting pattern. Assuming that the pith is at the centreline of the shape of the log 6 and there is no spiral grain it is therefore also possible to apply the same principles as in the previous example to measurement of only the shape. The availability of colour images may be used to improve detection of the knots 3 and possible outer spiral grain.

### Example 4

A different way of creating the three-dimensional virtual model 1 of the log 6 involves the use of one or more X-ray projections of the log 6, if necessary combined with knowledge of the outer shape.

In fact, starting with the X-ray images of the log it is possible to acquire further information; for example in Skog, Johan. Characterization of sawlogs using industrial X-ray and 3D scanning. Diss. Luleå tekniska universitet, 2013, there is an explanation of how to measure the density of wood after drying (4.3.2) and identify the presence of defects in the pith (4.3.4). Further works are also cited which explain how to estimate the knottiness and other characteristics of the log 6 starting from X-ray images and from the shape of the log 6.

Further information may also be extracted by using deep neural networks, which use those X-ray images and, if necessary, the outer shape of the log 6 as input data.

The computer-implemented method which is the main innovative aspect of this invention is advantageously applied in the context of industrial plants in which the logs 6 are cut into boards 2 and in which the boards 2 obtained in that way are inspected by a grading device 7 to receive a strength grade.

In particular, in accordance with a further innovative aspect of this invention, the method described so far may be used in the context of a more structured computer-implemented method, which allows selection of the cutting pattern to be used for cutting a log 6 into real wooden boards 2, in the context of a plant in which the real wooden boards 2 are then subjected to real strength grading by a known grading device 7.

In accordance with this application of the invention, the cutting pattern to be used for cutting a log 6 into real boards 2 is selected by estimating, before cutting, how the real boards 2 obtainable with each cutting pattern would be graded, then selecting the one which will allow the most advantageous overall result.

The method for selecting the cutting pattern comprises first the already described virtualisation step, during which the three-dimensional virtual model 1 of the log 6 to be cut is obtained.

The method also comprises an identifying step during which a plurality of candidate cutting patterns is identified which could be used for cutting the log 6. The candidate cutting patterns can be identified in any way suitable for the purpose, whether traditional or not.

In some embodiments all of the candidate cutting patterns are selected before proceeding with the steps described below. In other embodiments, in contrast, further candidate cutting patterns may be added even subsequently.

In some embodiments the candidate cutting patterns may be selected only in the context of several predetermined types (in particular regarding the board 2 formats which may be obtained). In other embodiments in contrast there may be more freedom of choice.

Each candidate cutting pattern is different from all of the others and comprises (advantageously is defined by) a set of cuttings planes whose positions relative to each other and whose position relative to the log 6 are known.

The method for selecting the cutting pattern then comprises execution of an assessing step for each candidate cutting pattern identified in the identifying step. During the assessing step, a prediction is made of the strength grade which the known grading device 7 would assign to the real wooden boards 2 if they were obtained from the log 6 using the candidate cutting pattern being considered.

In accordance with this invention, execution of the assessing step for assessing each candidate cutting pattern comprises, for each real wooden board 2 obtainable with the candidate cutting pattern applied to the log 6, execution of the above-described computer-implemented method for predicting the real strength grade which the known grading device 7 would assign to that real wooden board 2.

Finally, the method for selecting the cutting pattern comprises execution of a selecting step, during which there is selection of the chosen cutting pattern to be used to effectively cut the log 6, from the candidate cutting patterns for which the assessing step was carried out. Advantageously, the selecting step is performed based on a value of an objective function which takes into account the result of the assessing step carried out for each candidate cutting pattern. For example, the objective function may be a function which assigns to each board obtainable with the cutting pattern an economic value depending on the strength class estimated for it, and which calculates an economic value of the cutting pattern by adding together the economic values of all of the boards 2 obtainable.

In accordance with a further innovative aspect of this invention, the computer-implemented method for selecting the cutting pattern to be used to cut a log 6 into real wooden boards 2, may be used in the context of a log 6 cutting plant comprising a cutting device 8 which comprises one or more blades and is configured to cut a log 6 to obtain wooden boards 2, and a grading device 7, configured to analyse a wooden board cut from the log 6 and to assign it a real strength grade.

Advantageously, the plant also comprises a hardware infrastructure 9 comprising one or more computers, which is programmed to implement the method for selecting the cutting pattern, and a control system 10 which is operatively connected to the hardware infrastructure 9 and is configured to control the cutting device 8 in order to cut the log 6 according to the chosen cutting pattern supplied by the hardware infrastructure 9.

In some preferred embodiments, the plant also comprises one or more electronic analysis devices 11 configured to analyse one or more characteristics of the log 6 and to generate and save the digitally saved information about the log 6. Advantageously, those one or more electronic analysis devices 11 comprise one or more of the following: a tomographic device; an X-ray device; a three-dimensional scanner; a photographic scanner; a log 6 resonance frequency measuring device.

This invention brings important advantages.

In fact thanks to this invention it was possible to define a technical solution which allows a significant increase in the overall efficiency of a plant, that is to say, the logs being equal, it allows the obtainment of a greater number of boards which a known grading device can grade as boards suitable for structural use, thereby also maximising the economic value obtainable by the sawmill.

Finally, it should be noticed that this invention is relatively easy to produce and that even the cost linked to implementing the invention is not very high.

The invention described above may be modified and adapted in several ways without thereby departing from the scope of the inventive concept.

All details may be substituted with other technically equivalent elements and the materials used, as well as the shapes and dimensions of the various components, may vary according to requirements.

## Claims

1. A computer-implemented method for predicting a real strength grade which a known grading device (7) will assign to a real wooden board (2), before the real wooden board (2) is cut from a log (6) using a known cutting pattern, wherein the log (6) extends along a longitudinal axis and the known cutting pattern is configured to divide the log (6) into real boards (2) each having an end face which lies in a plane transversal to the longitudinal axis and lateral faces which are substantially parallel to the longitudinal axis, the known cutting pattern comprising a set of cutting planes which are substantially parallel to the longitudinal axis and which are arranged in known positions both relative to each other, and relative to the log (6), the method comprising the following operating steps:
a virtual cutting step, during which there is virtual cutting of a three-dimensional virtual model (1) of the log (6) previously generated by applying the known cutting pattern to the three-dimensional virtual model (1), and at the end of which a virtual board (4) is obtained which corresponds to the real wooden board (2) to be cut;
an assigning step, during which there is virtual adding to the virtual board (4) of surface and/or internal characteristics, obtaining them from digitally saved information about the log (6);
a virtual grading step, during which there is estimating of a virtual strength grade which the known grading device (7) would assign to a real board (2) if it were to have the characteristics of the virtual board (4) obtained at the end of the assigning step;
a predicting step, during which it is assumed that the real strength grade, which the known grading device (7) will assign to the real wooden board (2), corresponds to the virtual strength grade estimated in the virtual grading step.

2. The computer-implemented method according to claim 1, wherein the virtual grading step is carried out using an algorithm which correlates characteristics of the virtual board (4) with a virtual strength grade.

3. The computer-implemented method according to claim 2, wherein the virtual grading step is carried out by using as an algorithm a neural network or a deep neural network which uses virtual board (4) data as input data and which supplies the virtual strength grade as output data item.

4. The computer-implemented method according to claim 1, wherein the virtual grading step is carried out by simulating grading of the virtual board (4) by the known grading device (7).

5. The computer-implemented method according to claim 4, wherein the virtual grading step comprises a preparing step in which, for the virtual board (4), a set of virtual information to be assessed is obtained, where the virtual information to be assessed is of a same type as real information to be assessed based on which the known grading device (7) determines the real strength grade for a real board (2), and a processing step during which the set of virtual information to be assessed is processed with a processing algorithm used by the known grading device (7) for grading the strength of the real boards (2) starting with the set of real information to be assessed.

6. The computer-implemented method according to claim 5, wherein during the preparing step, for the virtual board (4), a set of virtual information to be assessed is generated which comprises one or more virtual X-ray images of the virtual board (4).

7. The computer-implemented method according to claim 5, wherein during the preparing step, for the virtual board (4), a set of virtual information to be assessed is generated which comprises one or more virtual maps which show a trend of wood fibres at one or more faces of the virtual board (4).

8. The computer-implemented method according to any one of claims 5 to 7, wherein the set of virtual information to be assessed also comprises a value linked to a resonance frequency of the log 6.

9. The computer-implemented method according to any one of claims 5 to 8, wherein the set of virtual information to be assessed is generated using the digitally saved information.

10. The computer-implemented method according to any one of claims 1 to 9, wherein the digitally saved information comprises one or more of the following: a tomographic model of the log (6); one or more X-ray views of the log (6); a three-dimensional geometric model of the outer shape of the log (6); a map of the colour of an outer lateral surface of the log (6).

11. The computer-implemented method according to claim 10, wherein the digitally saved information also comprises a value of a resonance frequency of the log 6.

12. The computer-implemented method according to any one of claims 1 to 11, also comprising a virtualisation step, during which the three-dimensional virtual model (1) of the log (6) is obtained.

13. A computer-implemented method for selecting a chosen cutting pattern to be used for cutting a log (6) into real wooden boards (2), in a plant in which the real wooden boards (2) are then subjected to real strength grading by a known grading device (7), wherein the log (6) extends along a longitudinal axis and the cutting pattern is configured to divide the log (6) into real boards (2) each having an end face which lies in a plane transversal to the longitudinal axis and lateral faces which are substantially parallel to the longitudinal axis, the cutting pattern comprising a set of cutting planes which are substantially parallel to the longitudinal axis and which are arranged in known positions both relative to each other, and relative to the log (6), the method comprising in order:
a virtualisation step, during which a three-dimensional virtual model (1) of the log (6) is obtained;
an identifying step during which a plurality of candidate cutting patterns is identified, each of which is different from all of the others and is defined by a set of cutting planes with known positions relative to each other and relative to the log (6); and
an assessing step carried out for each candidate cutting pattern identified in the identifying step, during which a prediction is made of a real strength grade which the known grading device (7) would assign to real wooden boards (2) if they were obtained from the log (6) using that candidate cutting pattern; and
a selecting step, during which there is selection of the chosen cutting pattern to be used to cut the log (6), from the candidate cutting patterns for which the assessing step was carried out;
wherein, during the assessing step for assessing each candidate cutting pattern, for each real wooden board (2) obtainable by cutting the log (6) with the candidate cutting pattern, the method comprises execution of the computer-implemented method according to any one of claims 1 to 11, for predicting the real strength grade which the known grading device (7) would assign to that real wooden board (2);
and wherein the selecting step is performed based on a value of an objective function which takes into account the result of the assessing step carried out for each candidate cutting pattern.

14. The computer-implemented method according to claim 13, wherein the virtualisation step is carried out, alternatively, either by creating the three-dimensional virtual model (1) of the log (6) using digitally saved information about geometric characteristics of the log (6), or by retrieving the three-dimensional virtual model (1) of the log (6) previously digitally saved.

15. The computer-implemented method according to claim 14, wherein the digitally saved information about the geometric characteristics of the log (6) comprises one or more of the following: a tomographic model of the log (6) and a three-dimensional geometric model of the outer shape of the log (6).

16. A computer program comprising instructions which, when executed by a hardware infrastructure (9) comprising one or more computers, cause the hardware infrastructure (9) to carry out a method according to any one of claims 1 to 15.

17. A data processing device comprising a hardware infrastructure (9) comprising one or more computers programmed to implement a method according to any one of claims 1 to 15.

18. A plant for cutting logs (6) comprising:
a cutting device (8) which comprises one or more blades and is configured to cut a log (6) to obtain real wooden boards (2);
a grading device (7) configured to analyse a real wooden board (2) cut from the log (6) and to assign it a real strength grade;
a hardware infrastructure (9) comprising one or more computers, which is programmed to implement the method according to any one of claims 13 to 15;
a control system (10) which is operatively connected to the hardware infrastructure (9) and is configured to control the cutting device (8) in order to cut the log (6) according to the chosen cutting pattern supplied by the hardware infrastructure (9).

19. The plant according to claim 18 also comprising one or more electronic analysis devices (11) configured to analyse one or more characteristics of the log (6) and to generate and save the digitally saved information about the log (6).

20. The plant according to claim 19, wherein the one or more electronic analysis devices (11) comprise one or more of the following: a tomographic device; an X-ray device; a three-dimensional scanner; a photographic scanner; a log 6 resonance frequency measuring device.
